# EUROPEAN PATENT APPLICATION

(11) **EP 3 231 366 A1**
(43) Date of publication of application: **18.10.2017**
(21) Application number: 15866754.3
(22) Date of filing: 11.12.2015
(51) Int. Cl.: A61B 5/053, A61B 5/0408

(54) **ELECTRODE TAPE**

(30) Priority: 12.12.2014 BR 102014031270
(71) Applicant: Timpel S.A., 05417-020 São Paulo - SP (BR)
(72) Inventor: HOLZHACKER, Rafael, 05417-020 São Paulo - SP (BR); CANDIANI, Igor Nowaski, 05711-000 São Paulo - SP (BR)
(74) Representative: Schwabe - Sandmair - Marx
(86) International application number: PCT/BR2015/050246
(87) International publication number: WO 2016/090450

(57) **Abstract**

The present invention is usually related to electrode belts and, more particularly, to electrode belts used to obtain signals from electrical impedance tomography. The electrode belt for acquiring signals of electrical impedance tomography, comprises at least one module (1), in which each module comprises at least two electrodes (3), wherein the center of each electrode (3) is placed at a predetermined distance (De) in relation to the center of at least one other adjacent electrode (3).

## Description

### FIELD OF THE INVENTION

The present invention relates generally to electrode belts and, particularly, to electrode belts used to obtain electrical impedance tomography (EIT) signals.

### BACKGROUND OF THE INVENTION

Within the techniques of image reconstruction known to the state of the art, 4 stand out: magnetic resonance imaging (MRI), CT Scan, medical ultrasound, as well as the electrical impedance tomography (EIT).

Although some image reconstruction techniques provide a better spatial resolution of images than the ones generated by electrical impedance tomography (EIT), the EIT has many advantages, such as: lower cost, high temporal resolution of images, not exposing the patient to radiation risks, and smaller sized equipment.

As a result, besides encouraging parsimony and safety, the techniques of electrical impedance tomography allow the equipment to easily be taken to the patient, so that the patient does not have to be moved from the bed to be examined.

The electrical impedance tomography (EIT) is an image acquisition technique, usually aimed at the thoracic region of the patient, based on the application of alternating electrical signals with frequencies from 10 kHz to 2.5 MHz, using electrodes attached to the patient's body surface.

Patients are any human being or animal. From this perspective, the above-mentioned techniques aimed at the thoracic region of the patient are generally related to the thorax, the area that extends from the base of the neck to the diaphragm of a human being or animal.

On the whole, the equipment used for this purpose comprises a plurality of electrodes placed in contact the skin. They are connected, by means of electrical conductors, to a processing unit that makes the mentioned alternating signal.

Preferably, the signals are injected through a first pair of electrodes selected from the plurality of electrodes, they run through the patient and, then, are acquired up by the other electrodes so the induced tension can be measured. Then, the previous procedure is repeated using a second pair of electrodes selected from the plurality of electrodes for the injection of the signal; following this sequence until all electrodes of the equipment have been selected, thus completing one exploring cycle.

The induced tensions that were acquired by the electrodes undergo a specific software treatment, allowing image generation, which usually represents the ventilation and perfusion phenomena in the organism observed.

The electrodes are usually held by a belt, which is placed around the body of the patient, preferably in the area of the thorax. In this regard, it is relevant to mention the document PI 0704408-9 that describes modular belts that have a plurality of electrodes, to be applied around a part of the body of a human or animal patient.

One of the known problems of the state of the art is related to the difficulty in setting up the electrodes on the patient, because it is very laborious and the patient has to be lifted from the bed.

Another problem known to the state of the art is related to pressure ulcers (wounds) on the patient's skin, caused by sensors and electrodes that are thick, of irregular surface, of variable thickness, that apply excessive and continuous pressure on the patient's skin, especially in situations where the patients are lying on or in direct contact with the thick parts of the belt, or even the cords that connect the belt to the equipment.

As seen, despite apparently being functional up to the present moment, the belts in the state of the art show some inconveniences and limitations concerning the electrode's attachment and placing.

Therefore, the present invention's objective is to provide an electrode belt for electrical impedance tomography that solves the problems known to the state of the art, in order to further improvements in the fixation and placing of the electrodes on the patient.

### DESCRIPTION OF THE INVENTION

In order to avoid the inconveniences of the state of the art and meet the goals above mentioned, among others, the invention is an electrode belt for acquiring signals of electrical impedance tomography that comprises at least one module, in which each module comprises at least two electrodes. The center of each electrode is placed at a predetermined distance (De) in relation to the center of at least one other adjacent electrode.

According to the additional or alternative embodiments of the invention, the following characteristics, alone or in possible technical combinations, may also be present:
- each module is of a predetermined size within a first, second, third, fourth, fifth or sixth size;
- the predetermined distance De for a first size module is from 19.3 mm to 21.3 mm;
- the predetermined distance De for a second size module is from 23.1 mm to 25.1 mm;
- the predetermined distance De for a third size module is from 25.9 mm to 27.9 mm;
- the predetermined distance De for a fourth size module is from 29.0 mm to 31.0 mm;
- the predetermined distance De for a fifth size module is from 32.4 mm to 34.4 mm;
- the predetermined distance De for a sixth size module is from 36.2 mm to 38.2 mm;
- the distance between the electrodes of each module is smaller than 10% of the perimeter of the patient's thorax;
- two modules attachable to the patient's body, wherein the distance between the electrode closer to one of the edges of the module and the electrode closer to one of the edges of the adjacent module is smaller than 10% of the perimeter of the patient's thorax;
- the distance between the electrodes on the patient's sternum area is smaller than 10% of the perimeter of the thorax.
- the contact resistance of each electrode is lower than 100 Ohms;
- each electrode's area is larger than 300 mm²;
- each electrode is coated with conductive silicone;
- the area between electrodes is coated with non-conductive silicone;
- the non-conductive silicone coating is even along the belt, wherein the level variation of the conductive and non-conductive silicone surfaces is smaller than 2 mm;
- each electrode is connected to a signal-conductor cable;
- it comprises a cable outlet placed in an area of the module located between half of the total length and one end of the belt;
- each module contains 16 electrodes;
- it comprises a cable outlet located in an area between the 5th and 8th electrode; and
- it comprises a cable outlet located in an area between the 9th and 12th electrode;
- the thickness of the belt is smaller than 6mm.

### BRIEF DESCRIPTION OF THE DRAWINGS

The objectives, advantages, technical and functional improvements of the invention will be better understood with the analysis of the description of preferred embodiments, made following with regard to the figures attached, which illustrate non-restrictive preferred embodiments, in which:
FIG. 1 shows a front elevational view of an electrode belt as the first embodiment of the invention;
FIG. 2 shows a front elevational view with a longitudinal sectional side view of the electrode of FIG. 1;
FIG. 3 shows a front elevational view of an electrode belt as the second embodiment of the invention; and
FIG. 4 shows a perspective view of a module's part of the electrode belt as the third embodiment of the invention.

### DESCRIPTION OF THE PREFERRED EMBODIMENT

The invention is now described regarding its preferred embodiments, referring to the attached figures. In the figures and description ahead, similar parts are marked with equal reference numbers. The figures are not, necessarily, in scale. That is, certain characteristics of the invention may be shown with exaggeration of scale or schematically, as well as some details of conventional elements may not be shown in order to illustrate this description in a more clear and concise way. The present invention is sensitive to embodiments in different ways. Specific embodiments are described in details and shown in the figures, with the understanding that the description must be considered an example of the principles here revealed, and the purpose is not to limit it only to what is illustrated and described in this descriptive report. We must acknowledge that the different teachings of the embodiments discussed next may be separately employed or in any appropriate combination to provide the same technical effects.

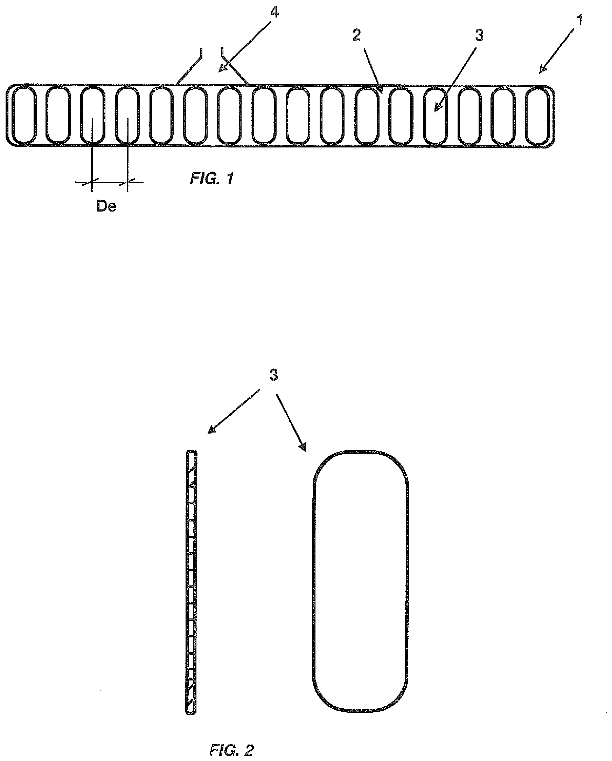

The present invention comprises an electrode belt, which, as it may be observed on FIG. 1, comprises a module 1 with 16 electrodes 3 placed equidistantly, that is, the distance from one electrode 3 to another adjacent is the same to any other electrode 3.

The electrode belt is particularly designed to acquire signals of electrical impedance tomography (EIT), therefore, the electrode belt is usually positioned in the area of the patient's thorax.

As it is known for those skilled in the art, the size and proportion of the belt should vary according to the size of the patient that will use it. Therefore, the present invention expects the use of at least five predetermined sizes, here named as: first size, second size, third size, fourth size, fifth size and sixth size. In a preferred embodiment, these sizes may have common names used in the market, such as: XS, S, M, L and XL

Thereby, the provisions of the present invention allow the use of the belt on a large selection of thorax's sizes, since the use of the appropriate sized belt, according to the patient's thorax, and the specific placement of the electrodes, ensure that the distance between the electrodes 3 will be a maximum of 10% of the thorax's size.

For example, the sizes of the present invention's belt may be determined as: the predetermined distance De for a first size module is from 19.3 mm to 21.3 mm; the predetermined distance De for a second size module is from 23.1 mm to 25.1 mm; the predetermined distance De for a third size module is from 25.9 mm to 27.9 mm; the predetermined distance De for a fourth size module is from 29.0 mm to 31.0 mm; the predetermined distance De for a fifth size module is from 32.4 mm to 34.4 mm; and the predetermined distance De for a sixth size module is from 36.2 mm to 38.2 mm.

In this particular process, the predetermined distance between the center of the 1st electrode and the center of the 16th electrode on a first size module is from 300 mm to 310 mm; the predetermined distance the predetermined distance between the center of the 1st electrode and the center of the 16th electrode on a second size module is from 357 mm to 367 mm; the predetermined distance between the center of the 1st electrode and the center of the 16th electrode on a third size module is from 399 mm to 409 mm; the predetermined distance between the center of the 1st electrode and the center of the 16th electrode on a fourth size module is from 445 mm to 455 mm; the predetermined distance between the center of the 1st electrode and the center of the 16th electrode on a fifth size module is from 496 mm to 506 mm; the predetermined distance between the center of the 1st electrode and the center of the 16th electrode on a sixth size module is from 553 mm to 563 mm;

In an embodiment of the invention, module 1 has a cable outlet 4 placed in an area of module 1 comprised between half of the total length and one end of the belt. This setting has the objective to allow the cable outlet 4 to stay between the patient's sternum and the axilla.

In other words, the placement of the cable outlet 4 is well set so that, when the belt is fastened on the patient, the cables 5 are closer to the sternum than to the spine of the patient, to facilitate access to the cables and avoid pressure ulcers caused by the patient's pressure when lying on the cables.

FIG. 1 shows an example of placement of the outlet 4 for signal transmission cable 5, which covers an area that comprises from the 5th to the 8th electrode.

FIG. 2 shows an electrode in accordance with an embodiment of the invention, wherein the area of the electrode 3 is larger than 300 mm², more specifically about 450 mm², represented by an element of 35 mm of length and 13 mm of width.

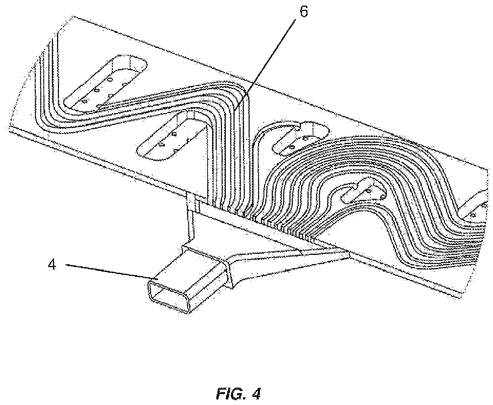

FIG. 4 illustrates another process of the invention, in which the non-conductive silicone has grooves 6 that work as ducts for the passage of cables 5 from electrodes 3 up to the cable outlet 4, so that the cables 5 are inside module 1, that is, the cables 5 are not in contact with the patient's skin when the belt is fastened.

In another embodiment of the invention, the belt consists of two modules 1, being the first module set with the cable outlet 4 placed between its half and left end, and the second module set with the cable outlet 4 placed between its half and right end, so that, when fastening the belt on the patient, the cable outlets 4 from both modules are placed on the thorax's front area. Particularly, modules 1 are placed contiguously along its longitudinal edges, around the patient's body, so that a module 1 does not overlap another and allowing all electrodes to be in contact with the patient, receiving electrical signals with no barriers. This setting of two modules allow the belt to be fastened on a patient without lifting him/her from the bed.

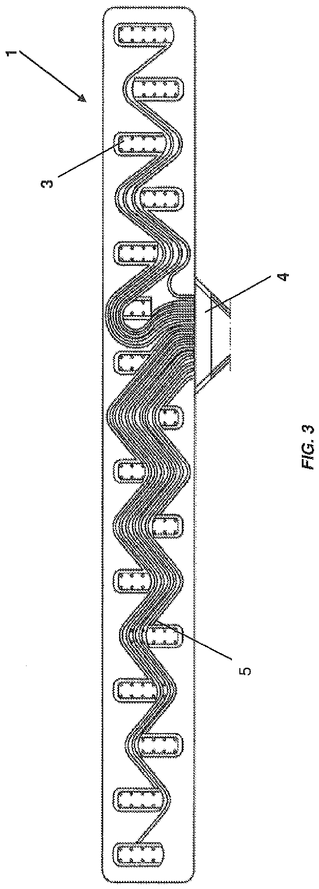

FIG. 3 illustrates another embodiment of the invention, in which a way of connecting the electrodes 3 to the outlet 4 of the signal transmission cable 5 is shown. The outlet 4 of the cable 5 is placed on the area that comprises electrodes 9th to 12th, being the module 1 set to be used on the left side of a patient, in a way that the outlet 4, and cables 5, are placed on the front area of the patient's thorax.

Particularly, the modules 1 are coated with silicone, wherein a conductive silicone is used on the area of the electrodes 3, so that the contact resistance is lower than 100 Ohms. In other areas, mainly on the area 2 between electrodes 3, the module is filled with non-conductive silicone.

Auspiciously, the non-conductive silicone is placed in a way that the belt's surface that will be in contact with the patient is flat and even, that is, with no variation between the level of the surfaces coated with the conductive silicone and the surfaces of the areas 2, covered with the non-conductive silicone.

In face of what was described above, it is visible that, auspiciously, the placement of the electrodes 3 in module 1 is done in a way that the distance 2 between two electrodes 3 is always smaller than 10% of the perimeter of the patient's thorax on the sternum area. This allows a uniform positioning and fixing of the electrodes on the patients, without leaving unwanted spaces between the electrodes, solving one of the existing problems with the state of the art.

Despite the invention being described regarding its preferred embodiments, it is understood that variations may occur in relation to what was described above without moving away from the scope of the invention. Consequently, the scope of protection is not limited to the embodiments described, but it is only limited by the following claims, which must be interpreted covering all its equivalents.

## Claims

1. An ELECTRODE BELT, for acquiring signals of electrical impedance tomography, **characterized by** the fact that it comprises at least one module (1), in which each module comprises at least two electrodes (3), wherein the center of each electrode (3) is placed at a predetermined distance (De) in relation to the center of at least one other adjacent electrode (3).

2. The ELECTRODE BELT of claim 1, wherein each module 1 is of a predetermined size within a first, second, third, fourth or fifth size.

3. The ELECTRODE BELT of claim 2, wherein the predetermined distance (De) may vary from:
19.3 mm to 21.3 mm, for a first size module;
23.1 mm to 25.1 mm, for a second size module;
25.9 mm to 27.9 mm, for a third size module;
29.0 mm to 31.0 mm, for a fourth size module;
32.4 mm to 34.4 mm, for a fifth size module; and
36.2 mm to 38.2 mm, for a sixth size module.

4. The ELECTRODE BELT of claims 1 to 3, wherein the distance between electrodes (3) is smaller than 10% of the perimeter of a patient's thorax.

5. The ELECTRODE BELT of claims 1 to 4, wherein it comprises two modules that can be fixed on the patient's body in a way that the distance (2) between the electrode (3) closer to an end of the adjacent module is smaller than 10% of the perimeter of the patient's thorax.

6. The ELECTRODE BELT of claim 5, wherein the modules (1) are placed contiguously along the longitudinal ends, in a way that a module (1) does not overlap another.

7. The ELECTRODE BELT of claims 1 to 6, wherein the resistance of contact of each electrode (3) is lower than 100 Ohms.

8. The ELECTRODE BELT of claims 1 to 7, wherein the area of each electrode is larger than 300 mm².

9. The ELECTRODE BELT of claims 1 to 8, wherein each electrode (3) is coated with conductive silicone.

10. The ELECTRODE BELT of claims 1 to 9, wherein the area (2) between electrodes (3) is filled with non-conductive silicone.

11. The ELECTRODE BELT of claim 10, wherein the non-conductive silicone filling is uniform throughout the belt, considering that the unevenness of the surface in the areas filled with conductive and non-conductive silicone is smaller than 2 mm.

12. The ELECTRODE BELT of claims 1 to 11, wherein each electrode is connected to a signal-conductive cable (5) in which each module has a cable outlet (4) placed on an area of the module between half of the total length and one end of the belt.

13. The ELECTRODE BELT of claims 1 to 12, wherein each module (1) has 16 electrodes (3).

14. The ELECTRODE BELT of claims 12 and 13, wherein the cable outlet (4) is placed on an area between the 5th and 8th electrode (3).

15. The ELECTRODE BELT of claims 12 and 13, wherein the cable outlet (4) is placed on an area between the 9th and 12th electrode (3).

16. The ELECTRODE BELT of claims 1 to 15, wherein the thickness of the belt is smaller than 6 mm.

17. The ELECTRODE BELT of claims 1 to 13, wherein the predetermined distance between the center of the 1st electrode and the center of the 16th electrode to a first size module is from 300 mm to 310 mm; the predetermined distance between the center of the 1st electrode and the center of the 16th electrode to a second size module is from 357 mm to 367 mm; the predetermined distance between the center of the 1st electrode and the center of the 16th electrode to a third size module is from 399 mm to 409 mm; the predetermined distance between the center of the 1st electrode and the center of the 16th electrode to a fourth size module is from 445 mm to 455 mm; the predetermined distance between the center of the 1st electrode and the center of the 16th electrode to a fifth size module is from 496 mm to 506 mm; the predetermined distance between the center of the 1st electrode and the center of the 16th electrode to a sixth size module is from 553 mm to 563 mm.
